# EUROPÄISCHE PATENTSCHRIFT

(11) **EP 1 562 896 B1**
(45) Veröffentlichungstag und Bekanntmachung des Hinweises auf die Patenterteilung: **02.02.2011**
(21) Anmeldenummer: 03773710.3
(22) Anmeldetag: 11.11.2003
(51) Int. Cl.: C07D 201/16, C07D 201/08

(54) **VERFAHREN ZUR REINIGUNG VON CAPROLACTAM**
METHOD FOR PURIFYING CAPROLACTAM
PROCEDE D'EPURATION DE CAPROLACTAME

(30) Priorität: 13.11.2002 DE 10253095
(43) Veröffentlichungstag der Anmeldung: 17.08.2005
(73) Patentinhaber: BASF SE, 67056 Ludwigshafen (DE)
(72) Erfinder: FISCHER, Rolf-Hartmuth, 69121 Heidelberg (DE); LUYKEN, Hermann, 67069 Ludwigshafen (DE); ANSMANN, Andreas, 69168 Wiesloch (DE); BASLER, Peter, 68519 Viernheim (DE); BENISCH, Christoph, 68165 Mannheim (DE); MAIXNER, Stefan, 68723 Schwetzingen (DE); MELDER, Johann-Peter, 67459 Böhl-Iggelheim (DE)
(86) Internationale Anmeldenummer: PCT/EP2003/012556
(87) Internationale Veröffentlichungsnummer: WO 2004/043914

(56) Entgegenhaltungen:
- EP-A- 0 022 161
- EP-A- 0 306 874
- EP-A- 0 311 960
- DE-A- 10 021 192
- DE-A- 10 021 199
- DE-A- 10 027 328
- US-A- 5 496 941
- US-A- 5 693 793

## Beschreibung

Die vorliegende Erfindung betrifft ein Verfahren zur Abtrennung von Hochsiedern aus einem Rohcaprolactam, das Hochsieder, Caprolactam und gegebenenfalls Leichtsieder enthält, und das erhalten wurde durch
a) Umsetzung von 6-Aminocapronitril mit Wasser zu einem Reaktionsgemisch
b) Abtrennung von Ammoniak und nicht umgesetztem Wasser aus dem Reaktionsgemisch unter Erhalt eines Rohcaprolactams,
   dadurch gekennzeichnet, dass man
c) das Rohcaprolactam einer Destillationsvorrichtung zuführt unter Erhalt
   eines ersten Teilstroms über Kopf als Produkt und
   eines zweiten Teilstroms über Sumpf,
   wobei man bei der Destillation
   den Druck so einstellt, dass eine Sumpftemperatur von 170°C nicht unterschritten wird, und
   den zweiten Teilstrom so einstellt, dass der Caprolactam-Gehalt des zweiten Teilstroms nicht weniger als 75 Gew.-%, bezogen auf den gesamten zweiten Teilstrom, beträgt, wobei man den zweiten Teilstrom aus Schritt c) teilweise oder vollständig in Schritt a) zurückführt.

Verfahren zur Herstellung von Caprolactam sind allgemein bekannt.

Es ist ebenfalls, beispielsweise aus Ullmann's Encyclopedia of Industrial Chemistry, 5. Ed., Vol. A5, VCH Verlagsgesellschaft mbH, Weinheim (Deutschland), 1986, Seite 46-48, oder Kirk-Othmer, Encyclopedia of Chemical Technology, 4. Ed., Vol. 4, John Wiley & Sons, New York, 1992, Seite 836, allgemein bekannt, daß Caprolactam, das für die Herstellung von Polymeren verwendet wird, eine Reinheit von 99,9 bis 99,94 % aufweisen muß, wobei die Hauptverunreinigung üblicherweise Wasser in einer Menge von 0,04 bis 0,1 % ist. Andere Verunreinigungen dürfen nur im Bereich von maximal wenigen ppm enthalten sein.
So kann Caprolactam durch Beckmann-Umlagerung von Cyclohexanonoxim mit Schwefelsäure oder Oleum hergestellt werden. Nach Neutralisation des auf diese Weise erhaltenen Gemischs mit Ammoniak kann das Caprolactam von dem als Nebenprodukt enstandenen Ammoniumsulfat durch Extraktion mit einem organischen Lösungsmittel erhalten werden.

In Abhängigkeit von den Verfahren zur Herstellung der zur Darstellung des Cyclohexanonoxims eingesetzten Edukte, wie Cyclohexanon und Hydroxylammoniumsulfat, den Oximierungs- und Umlagerungsbedingungen enthält das rohe Caprolactam, das durch Beckmann-Umlagerung erhalten wurde, Verunreinigungen, die sich in Art und Umfang unterscheiden. Typische Verunreinigungen von rohem Caprolactam, das durch Beckmann-Umlagerung hergestellt wurde, sind C-Methylcaprolactame, 6-Methylvalerolactam und n-Pentylacetamid.

Zur Reinigung des bei der Beckmann-Umlagerung erhaltenen Roh-Caprolactams sind verschiedene Verfahren beschrieben.

Gemäß DE-A-1253716 kann das Roh-Caprolactam durch Hydrierung in Suspension in Gegenwart eines Katalysators und unter Zusatz einer Säure gereinigt werden.

Gemäß DE-A-1253716 kann das Roh-Caprolactam durch Hydrierung in Suspension in Gegenwart eines Katalysators und unter Zusatz einer Base gereinigt werden.

DD-A-75083 beschreibt ein Verfahren zur Reinigung von Roh-Caprolactam, in dem das Roh-Caprolactam zunächst destilliert und anschließend, gelöst in einem organischen Lösungsmittel, in Gegenwart eines Katalysators hydriert und dann mit einem Ionentauscher behandelt wird.

Gemäß EP-A-411455 können die charakteristischen wichtigen Qualitätsmerkmale für Caprolactam eingehalten werden, indem man das Roh-Caprolactam kontinuierlich in einem Flüssigphasen-Verfahren hydriert.

Roh-Caprolactam, das durch Hydroformylierung von 3-Pentensäure und/oder ihren Estern zu 5-Formylvaleriansäure(estern) als Hauptprodukten und 4- und 3-Formylvaleriansäure(estern) als Nebenprodukten, extraktiver (WO 97/02228) oder destillativer (WO 97/06126) Abtrennung dieser verzweigten Formylvaleriansäure(ester), aminierender Hydrierung von 5-Formylvaleriansäure(estern) zu 6-Aminocapronsäure(estern) und/oder 6-Aminocapronsäureamid und Cyclisierung von 6-Aminocapronsäure(estern) oder 6-Aminocapronsäureamid erhalten wird, enthält andere typische Verunreinigungen.

So ist beispielsweise aus WO 99/48867, Beispiel 1, bekannt, ausgehend von 5-Formylvaleriansäureestern, nach WO 98/37063, Beispiel 9 aus Gemischen aus 6-Aminocapronsäure, 6-Aminocapronsäureamid und entsprechenden Oligomeren erhaltenes Rohcaprolactam unter Zusatz von 10 Gew.-% Wasser, zu kristallisieren. In diesem Rohcaprolactam, aus dem Hoch- und Leichtsieder vor der Kristallisation nicht abgetrennt wurden, waren 6345 ppm N-Methylcaprolactam, 100 ppm 5-Methylvalerolactam, 78 ppm Valeramid und andere Verunreinigungen enthalten. Die Rohcaprolactam/Wasser-Schmelze Verunreinigungen enthalten. Die Rohcaprolactam/Wasser-Schmelze wurde bei 50°C homogenisiert und dann auf 30°C abgekühlt. Die ausgefallenen Kristalle wurden abfiltriert und 2 bis 3 Mal mit wässrigem Caprolactam gewaschen. 5-Methylvalerolactam und Valeramid wurden auf 1 ppm, N-Methylcaprolactam auf 51 ppm abgereichert. Aus 73,6 g Rohlactam wurden 33,7 g Reinlactam erhalten (Caprolactam-Ausbeute: 45,8 %). Die Kennzahl der flüchtigen Basen (VB) wurde erst durch eine zweite Kristallisation erreicht. Wurden nach WO 99/48867, Beispiel 3, aus dem Rohcaprolactam vor der Kristallisation Hoch- und Leichtsieder abgetrennt, so betrug die Caprolactam-Ausbeute nach der Kristallisation 52 %.

Aus WO 99/65873 ist weiterhin bekannt, Caprolactam aus Gemischen mit 4-Ethyl-2-pyrrolidon, 5-Methyl-2-piperidon, 3-Ethyl-2-pyrrolidon und 3-Methyl-2-Piperidon oder Octahydrophenazin an Adsorptionsmitteln wie aktivierte Aktivkohle, Molekularsieben oder Zeolithen selektiv zu adsorbieren und nach Desorption reines Caprolactam zu erhalten. An diese Caprolactam-Abtrennung kann sich eine Schmelzkristallisation oder eine Kristallisation aus einem Lösungsmittel anschließen.

Es ist weiterhin bekannt, Roh-Caprolactam durch Kristallisation zu reinigen, das ausgehend von 6-Aminocapronitril nach WO 98/37063, Anspruch 8, zunächst mit Wasser zu 6-Aminocapronsäure hydrolysiert wird. Dann werden Wasser und durch Hydrolyse gebildeter Ammoniak abgetrennt, die gebildete 6-Aminocapronsäure wird cyclisiert und das dabei anfallende Roh-Caprolactam nach WO 99/48867 kristallisiert.

Caprolactam kann auch erhalten werden durch Reaktion von 6-Aminocapronitril ("ACN") mit Wasser in der Flüssigphase in der Gegenwart oder Abwesenheit eines Katalysators unter Freisetzung von Ammoniak.

Die bei dieser Reaktion erhaltene Mischung enthält neben Caprolactam, Wasser, Ammoniak, gegebenenfalls weiterem flüssigem Verdünnungsmittel Verunreinigungen mit einem Siedepunkt über dem von Caprolactam ("Hochsieder") und solche mit einem Siedepunkt unter dem von Caprolactam ("Leichtsieder").

Aus US-A-496,941, Beispiel, ist bekannt, daß nach der Abtrennung von Wasser, Lösungsmittel, Ammoniak, Leichtsieder und Hochsieder aus einer Mischung, erhalten bei der Umsetzung von ACN mit Wasser und Lösungsmittel, ein rohes Caprolactam mit einer Reinheit von 99,5 % erhalten wird.

Für ein Roh-Caprolactam, das aus ACN in der Flüssigphase erhalten wurde, sind andere Reinigungsverfahren beschrieben, da sich die Verunreinigungen eines solchen Roh-Caprolactams von denen eines Roh-Caprolactams, das durch andere Verfahren erhalten wurde, wie in US-A-5,496,941 beschrieben, deutlich unterscheiden.

Gemäß US-A-5,496,941 wird ACN in einem ersten Schritt in der Flüssigphase zu Caprolactam umgesetzt, Leichtsieder, Wasser, Ammoniak und gegebenenfalls weitere Lösungsmittel gleichzeitig abgetrennt, Hochsieder abgetrennt unter Erhalt eines Roh-Caprolactams in einer Reinheit von 99,5 %, dieses Roh-Caprolactam in Gegenwart eines Katalysators hydriert, das erhaltene Produkt mit einem sauren Ionentauscher oder Schwefelsäure behandelt und das erhaltene Produkt in Gegenwart einer Base destilliert.

Aus WO 96/20923 ist ein Verfahren zur Reinigung von Rohcaprolactam bekannt, das aus der Flüssigphasen-Cyclisierung von 6-Aminocapronitril mit Wasser in Gegenwart eines Lösungsmittels und von heterogenen Katalysatoren stammt. Hierbei wird Rohcaprolactam zunächst hydriert, dann mit sauren Agentien behandelt und zuletzt in Gegenwart von Alkali destilliert.

Nachteilig an diesen beiden Reinigungsverfahren ist, dass drei separate Reaktionsschritte für die Herstellung von Rein-Caprolactam benötigt werden.

Aus DE 100 21 199 A1 und DE 100 21 192 ist bekannt, durch Flüssig- oder Gasphasen-Cyclisierung gewonnenes Caprolactam nach Abtrennung von Ammoniak und Wasser durch Kristallisation zu reinigen.

Die genannten Verfahren zur Reinigung von Roh-Caprolactam, das aus ACN hergestellt wurde, weisen den Nachteil auf, daß sie technisch aufwendig und energieintensiv, insbesondere durch die zahlreichen Trennschritte, sind.

Der vorliegenden Erfindung lag die Aufgabe zugrunde, ein Verfahren zur Verfügung zu stellen, das die Herstellung von Caprolactam, das ausgehend von ACN erhalten wurde, in hoher Reinheit auf technisch einfache und energiesparende Weise ermöglicht.

Demgemäß wurde das eingangs definierte Verfahren gefunden.

In dem erfindungsgemäßen Verfahren setzt man ein Rohcaprolactam ein, das durch Umsetzung von 6-Aminocapronitril mit Wasser gemäß den Schritten a) und b) erhalten wurde.

Gemäß Schritt a) wird eine Mischung (I) enthaltend 6-Aminocapronitril, Wasser und gegebenenfalls flüssiges Verdünnungsmittel zu einer Mischung (II) enthaltend Caprolactam, Ammoniak, Wasser, gegebenenfalls flüssiges Verdünnungsmittel, Hochsieder und gegebenenfalls Leichtsieder, vorzugsweise in Gegenwart eines die Umsetzung katalytisch fördernden Feststoffes, umgesetzt.

Das für Schritt a) erforderliche ACN kann, wie aus Ullmann's Encyclopedia of Industrial Chemistry, 5. Ed., Vol. A5, VCH Verlagsgesellschaft mbH, Weinheim (Deutschland), 1986, Seite 46, Fig. 8, allgemein bekannt, aus Adipodinitril erhalten werden.
Besonders in Betracht kommt dabei die partielle katalytische Hydrierung von Adipodinitril in Gegenwart von Ammoniak als Lösungsmittel und beispielsweise als Suspensionskatalysator Rhodium auf Magnesiumoxid (US-A-4,601,859), Raney Nickel (US-A-2,762,835, WO 92/21650), Nickel auf Aluminiumoxid (US-A-2,208,598) oder als Festbettkatalysator Cu-Co-Zn-Spinell (DE-B-954416, US-A-2,257,814) oder Eisen (DE-A-42 35 466) oder ein Verfahren gemäß US-A-2,245,129, US-A-2,301,964, EP-A-150295, FR-A-2 029 540 oder einem in US-A-5,496,941 beschriebenen Verfahren.

Das für diese Umsetzung erforderliche Adipodinitril wird technisch hergestellt, beispielsweise durch doppelte Hydrocyanierung von Butadien in Gegenwart von Nickel enthaltenden Katalysatoren, und ist kommerziell beispielsweise über die Firma Aldrich-Chemie Gesellschaft mbH & Co. KG, Steinheim, Deutschland verfügbar.

Die Umsetzung von Mischung (I) zu Mischung (II) kann gemäß US-A-4,628,085 in der Gasphase an Kieselgel bei 300°C erfolgen.
Ebenso kann diese Umsetzung gemäß US-A-4,625,023 in der Gasphase an einem Kieselgel-oder Kupfer/Chrom/Barium-Titanoxid-Katalysator durchgeführt werden. Gemäß FR-A-2029540 kann die Umsetzung in Gegenwart von Katalysatoren durchgeführt werden, wobei als Katalysatoren metallisches Zn oder Cu-Pulver oder Oxide, Hydroxide, Halogenide, Cyanide des Rubidiums, Bleis, Quecksilbers oder der Elemente mit einer Ordnungszahl 21 bis 30 oder 39 bis 48 Verwendung finden. Die beschriebenen Katalysatoren werden in diskontinuierlich betriebenen Rührautoklaven als Suspensionskatalysatoren eingesetzt.

Die Umsetzung von Mischung (I) zu Mischung (II) kann auch beispielsweise gemäß EP-A-659 741, WO 96/22974, DE 19632006 , WO 99/47500 oder WO 99/28296 erfolgen.

Vorzugsweise kann die Umsetzung in der Gasphase bei Temperaturen von im allgemeinen 200 bis 550°C, vorzugsweise 250 bis 400°C, durchgeführt werden; der Druck liegt im allgemeinen im Bereich von 0,01 bis 10 bar, vorzugsweise bei Normaldruck, wobei darauf zu achten ist, daß das Reaktionsgemisch unter den angewandten Bedingungen zum überwiegenden Teil gasförmig ist.

Die Katalysatorbelastungen betragen üblicherweise 0,05 bis 2, vorzugsweise 0,1 bis 1,5, insbesondere 0,2 bis 1 kg 6-Aminocapronitril pro Liter Katalysatorvolumen pro Stunde.

Die Umsetzung kann diskontinuierlich, vorzugsweise kontinuierlich durchgeführt werden.

Als Reaktoren kommen vorteilhaft solche in Betracht, wie sie im allgemeinen für Gasphasenreaktionen an bewegten oder stationären Feststoff-Katalysatoren bekannt sind. Vorzugsweise können ein Wirbelbettreaktor, vorzugsweise Festbett-Reaktor, wie ein Horden-Reaktor, insbesondere ein Röhrenreaktor, eingesetzt werden. Es sind auch Kombinationen solcher Reaktoren möglich.

Pro mol ACN werden im allgemeinen 1 bis 50, vorzugsweise 1 bis 10 mol Wasser eingesetzt.

Die Mischung (I) kann auch weitere organische Verbindungen enthalten, die unter den Reaktionsbedingungen gasförmig vorliegen, wie Alkohole, Amine oder aromatische oder aliphatische Kohlenwasserstoffe.

Als katalytisch aktive Verbindungen der Katalysatoren können beispielsweise Siliciumdioxid als pyrogen hergestelltes Siliciumdioxid, als Kieselgel, Kieselgur, Quarz oder Mischungen derselben, Kupferchromit, vorzugsweise Aluminiumoxid, Titanoxid, vorzugsweise Titandioxid, Lanthanphosphate, Lanthanoxide in Betracht wie auch Gemische solcher Verbindungen.

Aluminiumoxid ist in allen Modifikationen, die durch Erhitzen der Vorläuferverbindungen Aluminiumhydroxid (Gibbsit, Böhmit, Pseudo-Böhmit, Bayerit und Diaspor) bei unterschiedlichen Temperaturen erhalten werden können, geeignet. Dazu gehören insbesondere gamma- und alpha-Aluminiumoxid und deren Gemische.

Titandioxid ist amorph und in allen seinen Modifikationen, vorzugsweise Anatas und Rutil, sowie Mischungen solcher Modifikationen geeignet.

Lanthanphosphate sind in ihren verschiedenen Modifikationen, stöchiometrischen Verhältnissen zwischen Lanthan und Phosphateinheit und Kondensationsgraden der Phophateinheiten (Monophosphat, Oligophosphate wie Diphosphate oder Triphosphate, Polyphosphate) einzeln oder im Gemisch geeignet.

Diese Verbindungen können in Form von Pulvern, Gries, Splitt, Strängen oder zu Tabletten gepreßt, verwendet werden. Die Form der Verbindungen richtet sich in der Regel nach den Erfordernissen der jeweiligen Reaktionsführung, wobei in Wirbelbettfahrweise vorteilhaft Pulver oder Gries verwendet wird. Bei der Festbettfahrweise werden üblicherweise Tabletten oder Stränge mit Durchmessern zwischen 1 mm und 6 mm verwendet.

Die Verbindungen können in reiner Form (Gehalt des jeweiligen Verbindungen > 80 Gew.-%), als Gemisch der oben genannten Verbindungen, wobei die Summe der oben genannten Verbindungen > 80 Gew.-% betragen soll, oder als Trägerkatalysator, wobei die oben genannten Verbindungen auf einen mechanisch und chemisch stabilen Träger meist mit hoher Oberfläche aufgebracht werden können, verwendet werden.

Die reinen Verbindungen können durch Fällung aus wäßrigen Lösungen hergestellt worden sein, z.B. Titandioxid nach dem Sulfatprozeß oder durch andere Verfahren wie die pyrogene Herstellung von feinen Aluminiumoxid-, Titandioxid- oder Zirkondioxid-Pulvern, die käuflich zu erhalten sind.

Zur Herstellung von Gemischen der verschiedenen Verbindungen stehen mehrere Methoden zur Wahl. Die Verbindungen oder deren Vorläuferverbindungen, die durch Calzinieren in die Oxide umwandelbar sind, können z.B. durch eine gemeinsame Fällung aus Lösung hergestellt werden. Dabei wird im allgemeinen eine sehr gute Verteilung der beiden verwendeten Verbindungen erhalten. Die Verbindungs- oder Vorläufergemische können auch durch eine Fällung der einen Verbindung oder Vorläufers in Gegenwart der als Suspension von fein verteilten Teilchen vorliegenden zweiten Verbindung oder Vorläufers ausgefällt werden. Eine weitere Methode besteht im mechanischen Mischen der Verbindungs- oder Vorläuferpulver, wobei dieses Gemisch als Ausgangsmaterial zur Herstellung von Strängen oder Tabletten Verwendung finden kann.

Zur Herstellung von Trägerkatalysatoren bieten sich im Prinzip alle in der Literatur beschriebenen Methoden an. So können die Verbindungen in Form ihrer Sole durch einfaches Tränken auf dem Träger aufgebracht werden. Durch Trocknen und Calzinieren werden die flüchtigen Bestandteile des Sols üblicherweise aus dem Katalysator entfernt. Solche Sole sind für Titandioxid und Aluminiumoxid käuflich erhältlich.

Eine weitere Möglichkeit zum Aufbringen von Schichten der katalytisch aktiven Verbindungen besteht in der Hydrolyse oder Pyrolyse von organischen oder anorganischen Verbindungen. So kann ein keramischer Träger mit Titandioxid durch Hydrolyse von Titan-Isopropylat oder anderen Ti-Alkoxiden in dünner Schicht belegt werden. Weitere geeignete Verbindungen sind unter anderen TiC14 und , Aluminiumnitrat. Geeignete Träger sind Pulver, Stränge oder Tabletten der genannten Verbindungen selbst oder anderer stabiler Verbindungen wie Steatit oder Siliciumcarbid. Die verwendeten Träger können zur Verbesserung des Stofftransports makroporös ausgestaltet sein.

Die Reaktion kann in Gegenwart eines hinsichtlich der Umsetzung von Mischung (I) zu Mischung (II) inerten Gases, vorzugsweise Argon, insbesondere Stickstoff, durchgeführt werden. Das Volumenverhältnis des inerten Gases zu dem unter den Reaktionsbedingungen gasförmigen ACN kann vorteilhaft bis zu 100 betragen.

Als besonders bevorzugt kommt als Schritt 1) ein Verfahren in Betracht, wie es in US-A-5,646,277 oder US-A-5,739,324 beschrieben ist.

Bei diesen Verfahren wird die Umsetzung in flüssiger Phase bei Temperaturen von im allgemeinen 140 bis 320°C, vorzugsweise 160 bis 280°C, durchgeführt; der Druck liegt im allgemeinen im Bereich von 1 bis 250 bar, vorzugsweise von 5 bis 150 bar, wobei darauf zu achten ist, daß das Reaktionsgemisch unter den angewandten Bedingungen zum überwiegenden Teil flüssig ist. Die Verweilzeiten liegen im allgemeinen im Bereich von 1 bis 120, vorzugsweise 1 bis 90 und insbesondere 1 bis 60 min. In einigen Fällen haben sich Verweilzeiten von 1 bis 10 min als völlig ausreichend erwiesen.
Die Umsetzung kann diskontinuierlich, vorzugsweise kontinuierlich durchgeführt werden. Als Reaktor kommt ein Rührkessel, Autoklav, vorzugsweise ein Festbett-Röhrenreaktor, in Betracht. Es sind auch Kombinationen solcher Reaktoren möglich.

Pro mol ACN werden im allgemeinen mindestens 0,1 mol, vorzugsweise 0,5 bis 100 und insbesondere 1 bis 20 mol Wasser eingesetzt.

Vorteilhaft wird das ACN in Form einer 1 bis 50 gew.-%igen, insbesondere 5 bis 50 gew.-%igen, besonders vorzugsweise 5 bis 30 gew.-%igen Lösung in Wasser, wobei dann das Lösungsmittel gleichzeitig Reaktionspartner ist, oder in Gemischen enthaltend Wasser und ein flüssiges Verdünnungsmittel eingesetzt. Als flüssiges Verdünnungsmittel seien beispielhaft Alkanole wie Methanol, Ethanol, n- und i-Propanol, n-, i- und t-Butanol und Polyole wie Diethylenglykol und Tetraethylenglykol, Kohlenwasserstoffe wie Petrolether, Benzol, Toluol, Xylol, Lactame wie Pyrrolidon oder Caprolactam oder alkylsubstituierte Lactame wie N-Methylpyrrolidon, N-Methylcaprolactam oder N-Ethylcaprolactam sowie Carbonsäureester, vorzugsweise von Carbonsäuren mit 1 bis 8 C-Atomen genannt. Auch Ammoniak kann bei der Reaktion anwesend sein. Selbstverständlich können auch Mischungen organischer flüssiger Verdünnungsmittel Anwendung finden. Mischungen aus Wasser und Alkanolen im Gewichtsverhältnis Wasser/Alkanol 1-75/25-99, vorzugsweise 1-50/50-99 haben sich in einigen Fällen als besonders vorteilhaft herausgestellt.

Es ist prinzipiell genauso möglich, ACN als Reaktand und gleichzeitig Lösungsmittel anzuwenden.

In einer besonders bevorzugten Ausführungsform kommen solche flüssigen Verdünnungsmittel in Betracht, die unter bestimmten Druck, Temperatur und Konzentrationsbedingungen mit Wasser eine Mischungslücke aufweisen. Unter einer Mischungslücke wird dabei die Auftrennung der Mischung in zwei flüssige Phasen verstanden, wobei eine der beiden Phasen einen höheren Gewichts-Anteil an Wasser, bezogen auf die Summe aus Wasser und flüssigem Verdünnungsmittel, aufweist als die andere Phase. Besonders hierfür geeignete flüssige Verdünnungsmittel sind Kohlenwasserstoffe, wie Benzol, Toluol oder Xyxlol, insbesondere Toluol.

Die Umsetzung gemäß Schritt a) kann dabei in der Mischungslücke, also in Gegenwart von zwei flüssigen Phasen, oder vorzugsweise außerhalb der Mischungslücke, also in Gegenwart einer flüssigen Phase durchgeführt werden.

Als heterogene Katalysatoren können beispielsweise verwendet werden: Saure, basische oder amphotere Oxide der Elemente der zweiten, dritten oder vierten Hauptgruppe des Periodensystems, wie Calciumoxid, Magnesiumoxid, Boroxid, Aluminiumoxid, Zinn-Oxid oder Siliciumdioxid als pyrogen hergestelltes Siliciumdioxid, als Kieselgel, Kieselgur, Quarz oder Mischungen derselben, weiterhin Oxide von Metallen der zweiten bis sechsten Nebengruppe des Periodensystems, wie Titanoxid, amorph, als Anatas oder Rutil, Zirkonoxid, Zinkoxid, Manganoxid oder Mischungen davon. Ebenfalls verwendbar sind Oxide der Lanthaniden und Aktiniden, wie Ceroxid, Thoriumoxid, Praseodymoxid, Samariumoxid, Seltenerd-Mischoxid, oder Mischungen davon mit zuvor genannten Oxiden. Weitere Katalysatoren können beispielsweise sein:
Vanadiniumoxid, Nioboxid, Eisenoxid, Chromoxid, Molybdänoxid, Wolframoxid oder Mischungen davon. Mischungen der genannten Oxide untereinander sind ebenfalls möglich. Auch einige Sulfide, Selenide und Telluride wie Zink-Tellurid, Zinn-Selenid, Molybdänsulfid, Wolframsulfid, Sulfide des Nickels, Zinks und Chroms sind einsetzbar.

Die vorstehend genannten Verbindungen können mit Verbindungen der 1. und 7. Hauptgruppe des Periodensystems dotiert sein bzw. diese enthalten.

Weiterhin sind Zeolithe, Phosphate und Heteropolysäuren, sowie saure und alkalische lonenaustauscher wie beispielsweise Nafion als geeignete Katalysatoren zu nennen.

Gegebenenfalls können diese Katalysatoren bis zu jeweils 50 Gew.-% an Kupfer, Zinn, Zink, Mangan, Eisen, Kobalt, Nickel, Ruthenium, Palladium, Platin, Silber oder Rhodium enthalten.

Als besonders bevorzugte Katalysatoren, die unter den oben beschriebenen Reaktionsbedingungen sehr hohe Umsätze, Ausbeuten, Selektivitäten und Standzeiten besitzen, kommen heterogene Katalysatoren auf Basis Titanoxid, Zirkonoxid, Ceroxid und Aluminiumoxid in Betracht. Diese können in Form von Pulvern, Gries, Splitt, Strängen oder zu Tabletten gepreßt, verwendet werden. Die Form der Oxide richtet sich in der Regel nach den Erfordernissen der jeweiligen Reaktionsführung, wobei in Suspension Pulver oder Gries verwendet wird. Bei der Festbettfahrweise werden üblicherweise Tabletten oder Stränge mit Durchmessern zwischen 1 mm und 10 mm verwendet.

Aluminiumoxid ist in allen Modifikationen, die durch Erhitzen der Vorläuferverbindungen Aluminiumhydroxid (Gibbsit, Böhmit, Pseudo-Böhmit, Bayerit und Diaspor) bei unterschiedlichen Temperaturen erhalten werden können, geeignet. Dazu gehören insbesondere gamma- und alpha-Aluminiumoxid und deren Gemische.

Die Oxide können in reiner Form (Gehalt des jeweiligen Oxids > 80 Gew.-%), als Gemisch der oben genannten Oxide, wobei die Summe der oben genannten Oxide > 80 Gew.-% betragen soll, oder als Trägerkatalysator, wobei die oben genannten Oxide auf einen mechanisch und chemisch stabilen Träger meist mit hoher Oberfläche aufgebracht werden können, verwendet werden.

Die reinen Oxide können durch Fällung aus wäßrigen Lösungen hergestellt worden sein, z.B. Titandioxid nach dem Sulfatprozeß oder durch andere Verfahren wie die pyrogene Herstellung von feinen Aluminiumoxid-, Titandioxid- oder Zirkondioxid-Pulvern, die käuflich zu erhalten sind.

Zur Herstellung von Gemischen der verschiedenen Oxide stehen mehrere Methoden zur Wahl. Die Oxide oder deren Vorläuferverbindungen, die durch Calzinieren in die Oxide umwandelbar sind, können z.B. durch eine gemeinsame Fällung aus Lösung hergestellt werden. Dabei wird im allgemeinen eine sehr gute Verteilung der beiden verwendeten Oxide erhalten. Die Oxid- oder Vorläufergemische können auch durch eine Fällung des einen Oxids oder Vorläufers in Gegenwart des als Suspension von fein verteilten Teilchen vorliegenden zweiten Oxids oder Vorläufers ausgefällt werden. Eine weitere Methode besteht im mechanischen Mischen der Oxid- oder Vorläuferpulver, wobei dieses Gemisch als Ausgangsmaterial zur Herstellung von Strängen oder Tabletten Verwendung finden kann.

Zur Herstellung von Trägerkatalysatoren bieten sich im Prinzip alle in der Literatur beschriebenen Methoden an. So können die Oxide in Form ihrer Sole durch einfaches Tränken auf dem Träger aufgebracht werden. Durch Trocknen und Calzinieren werden die flüchtigen Bestandteile des Sols üblicherweise aus dem Katalysator entfernt. Solche Sole sind für Titandioxid, Aluminiumoxid und Zirkondioxid käuflich erhältlich.

Eine weitere Möglichkeit zum Aufbringen von Schichten der aktiven Oxide besteht in der Hydrolyse oder Pyrolyse von organischen oder anorganischen Verbindungen. So kann ein keramischer Träger mit Titandioxid durch Hydrolyse von Titan-Isopropylat oder anderen Ti-Alkoxiden in dünner Schicht belegt werden. Weitere geeignete Verbindungen sind unter anderen TiCl₄, Zirkonylchlorid, Aluminiumnitrat und Cernitrat. Geeignete Träger sind Pulver, Stränge oder Tabletten der genannten Oxide selbst oder anderer stabiler Oxide wie Siliciumdioxid. Die verwendeten Träger können zur Verbesserung des Stofftransports makroporös ausgestaltet sein.

Gemäß Schritt b) wird aus Mischung (II) Ammoniak und nicht umgesetztes Wasser entfernt unter Erhalt einer Mischung (III) enthaltend Caprolactam, gegebenenfalls flüssiges Verdünnungsmittel, Hochsieder und gegebenenfalls Leichtsieder.

Die Abtrennung des Ammoniaks aus Mischung (II) kann prinzipiell nach an sich für die Stofftrennung bekannten Verfahren, wie Extraktion oder vorzugsweise Destillation, oder eine Kombination solcher Verfahren erfolgen.

Die Destillation kann man vorteilhaft bei Sumpftemperaturen von 60 bis 220°C, insbesondere von 100 bis 220°C durchführen. Dabei stellt man üblicherweise einen Druck, gemessen am Kopf der Destillationsverrichtung von 2 bis 30 bar absolut ein.

Für die Destillation kommen hierfür übliche Apparaturen in Betracht, wie sie beispielsweise in: Kirk-Othmer, Encyclopedia of Chemical Technology, 3.Ed., Vol. 7, John Wiley & Sons, New York, 1979, Seite 870-881 beschrieben sind, wie Siebbodenkolonnen, Glockenbodenkolonnen, Packungskolonnen oder Füllkörperkolonnen.

Die Destillation kann man in mehreren, wie 2 oder 3 Kolonnen, vorteilhaft einer einzigen Kolonne durchführen.

Gemäß Schritt b) werden aus Mischung (III) Wasser ganz oder teilweise und gegebenenfalls flüssige Verdünnungsmittel entfernt unter Erhalt eines Rohcaprolactams (IV) enthaltend Caprolactam, Hochsieder und gegebenenfalls Leichtsieder.

Wurde in Schritt a) ein flüssiges Verdünnungsmittel eingesetzt, können Wasser und flüssiges Verdünnungsmittel in Schritt b) gleichzeitig oder das Wasser vor oder nach dem flüssigen Verdünnungsmittel abgetrennt werden.

Die Abtrennung des Wassers und gegebenenfalls des flüssigen Verdünnungsmittels aus Mischung (III) kann prinzipiell nach an sich für die Stofftrennung bekannten Verfahren, wie Extraktion, Kristallisation oder vorzugsweise Destillation, oder eine Kombination solcher Verfahren erfolgen.

Die Destillation kann man vorteilhaft bei Sumpftemperaturen von 50 bis 250°C, insbesondere von 100 bis 230°C durchführen.

Für die Destillation kommen hierfür übliche Apparaturen in Betracht, wie sie beispielsweise in: Kirk-Othmer, Encyclopedia of Chemical Technology, 3.Ed., Vol. 7, John Wiley & Sons, New York, 1979, Seite 870-881 beschrieben sind, wie Siebbodenkolonnen, Glockenbodenkolonnen, Packungskolonnen oder Füllkörperkolonnen.

Besonders bevorzugt ist eine wärmegekoppelte mehrstufige Abtrennung des Wassers und gegebenenfalls des flüssigen Verdünnungsmittels.

Wurde gemäß einer besonders bevorzugten Ausführungsform Schritt a) in Gegenwart eines flüssigen Verdünnungsmittels durchgeführt, das mit Wasser eine Mischungslücke aufweist, so kann die Abtrennung des Wassers vorteilhaft erfolgen, indem man das System, insbesondere bei Durchführung von Schritt a) in einer einzigen flüssigen Phase, in diese Mischungslücke überführt unter Ausbildung von zwei flüssigen Phasen, von denen eine Phase einen höheren Gewichts-Anteil an Wasser, bezogen auf die Summe aus Wasser und flüssigem Verdünnungsmittel, aufweist als die andere Phase, und dann die Phase mit dem höheren Gewichts-Anteil an Wasser von der anderen Phase, die üblicherweise den überwiegenden Gehalt an Rohcaprolactam enthält, abtrennt.

Das flüssige Verdünnungsmittel kann von der den überwiegenden Gehalt an Rohcaprolactam aufweisende Phase dann nach an sich bekannten Verfahren, beispielsweise wie bereits oben beschrieben, abgetrennt werden.

Vor der Zuführung des Rohcaprolactams (IV) in Schritt c) kommt in einer bevorzugten Ausführungsform zwischen den Schritten b) und c) die Abtrennung von Leichtsieder aus dem Roh-caprolactam (IV) in Betracht.

Die Abtrennung von Leichtsieder kann prinzipiell nach an sich für die Stofftrennung bekannten Verfahren, wie Extraktion, Kristallisation oder vorzugsweise Destillation, oder eine Kombination solcher Verfahren verfolgen.

Die Destillation kann man vorteilhaft bei Sumpftemperaturen von 50 bis 250°C, insbesondere von 100 bis 230°C durchführen. Dabei stellt sich üblicherweise ein Druck, gemessen am Kopf der Destillationsverrichtung von 1 bis 500, vorzugsweise 5 bis 100 mbar absolut ein.

Für die Destillation kommen hierfür übliche Apparaturen in Betracht, wie sie beispielsweise in: Kirk-Othmer, Encyclopedia of Chemical Technology, 3.Ed., Vol. 7, John Wiley & Sons, New York, 1979, Seite 870-881 beschrieben sind, wie Siebbodenkolonnen, Glockenbodenkolonnen, Packungskolonnen oder Füllkörperkolonnen.

Die Destillation zur Abtrennung der Leichtsieder kann man in mehreren, wie 2 oder 3 Kolonnen, vorteilhaft einer einzigen Kolonne durchführen.
Die Destillation zur Abtrennung der Hochsieder kann man in mehreren, wie 2 oder 3 Kolonnen, vorteilhaft einer einzigen Kolonne durchführen.

Als Leichtsieder kommt bei dieser Abtrennung insbesondere 6-Aminocapronitril in Betracht.

In einer weiteren bevorzugten Ausführungsform kommt die Abtrennung von Leichtsieder aus dem Produktstrom nach Schritt c) in Betracht.

Die Abtrennung von Leichtsieder kann prinzipiell nach an sich für die Stofftrennung bekannten Verfahren, wie Extraktion, Kristallisation oder vorzugsweise Destillation, oder eine Kombination solcher Verfahren verfolgen.

Die Destillation kann man vorteilhaft bei Sumpftemperaturen von 50 bis 250°C, insbesondere von 100 bis 230°C durchführen. Dabei stellt sich üblicherweise ein Druck, gemessen am Kopf der Destillationsverrichtung von 1 bis 500, vorzugsweise 5 bis 100 mbar absolut ein.

Für die Destillation kommen hierfür übliche Apparaturen in Betracht, wie sie beispielsweise in: Kirk-Othmer, Encyclopedia of Chemical Technology, 3.Ed., Vol. 7, John Wiley & Sons, New York, 1979, Seite 870-881 beschrieben sind, wie Siebbodenkolonnen, Glockenbodenkolonnen, Packungskolonnen oder Füllkörperkolonnen.

Die Destillation zur Abtrennung der Leichtsieder kann man in mehreren, wie 2 oder 3 Kolonnen, vorteilhaft einer einzigen Kolonne durchführen.

Die Destillation zur Abtrennung der Hochsieder kann man in mehreren, wie 2 oder 3 Kolonnen, vorteilhaft einer einzigen Kolonne durchführen.

Als Leichtsieder kommt bei dieser Abtrennung insbesondere 6-Aminocapronitril in Betracht.

Das Rohcaprolactam wird erfindungsgemäß einer Destillationsvorrichtung K1 zugeführt.

In dieser Destillationsvorrichtung wird ein erster Teilstrom über Kopf erhalten. Dieser Teilstrom enthält im wesentlichen gereinigtes Caprolactam als Produkt.

Weiterhin wird in dieser Destillationsvorrichtung ein zweiter Teilstrom über Sumpf erhalten. Dieser Teilstrom enthält Caprolactam und Hochsieder.

Als Destillationsvorrichtung kommen hierfür übliche Apparaturen in Betracht, wie sie beispielsweise in: Kirk-Othmer, Encyclopedia of Chemical Technology, 3.Ed., Vol. 7, John Wiley & Sons, New York, 1979, Seite 870-881 beschrieben sind, wie Siebbodenkolonnen, Glockenbodenkolonnen, Packungskolonnen oder Füllkörperkolonnen.

Die Destillation kann man in mehreren, wie 2 oder 3 Kolonnen, vorteilhaft einer einzigen Kolonne durchführen.

Setzt man als erste Destillationsvorrichtung eine Kolonne ein, so kann diese Kolonne vorteilhaft 1 bis 30 theoretische Böden, insbesondere 5 bis 20 theoretische Böden aufweisen.

In einer bevorzugten Ausführungsform kann man Rohcaprolactam (IV) auf den Kopf der Destillationsvorrichtung K1 aufgeben.

Erfindungsgemäß stellt man in der Destillationsvorrichtung K1 den Druck so ein, dass eine Sumpftemperatur von 170°C, vorzugsweise von 185 C nicht unterschritten wird.
Vorteilhaft kommt eine Sumpftemperatur von höchstens 300°C, insbesondere höchstens 250°C in Betracht.

Weiterhin stellt man den zweiten Teilstrom so ein, dass der Caprolactam-Gehalt des zweiten Teilstroms nicht weniger als 75 Gew.-%, bezogen auf den gesamten zweiten Teilstrom, beträgt.

Die für die Erzielung der erfindungsgemäßen Maßgaben optimalen Parameter für den Betrieb der gewählten Destillationsvorrichtung können dabei leicht durch wenige einfache Vorversuche ermittelt werden.

Weiterhin wird der zweite Teilstrom teilweise oder vollständig in Schritt a) zurück geführt.

Es war überraschend, dass es erfindungsgemäß gelingt, aus ACN hergestelltes Roh-Caprolactam destillativ auf technisch einfache und wirtschaftliche Weise in Caprolactam umzuwandeln, aus dem Polycaprolactam hergestellt werden kann.

### Beispiele

### Beispiel 1

105 kg/h Rohcaprolactam, das durch Umsetzung von 6-Aminocapronitril mit Wasser erhalten wurde, wurden einer Destillationskolonne mit 5 theoretischen Trennstufen zugeführt.
Über Kopf wurden 85 kg/h Caprolactam abgezogen.
Über Sumpf wurden 20 kg/h Caprolactam mit einem Hochsiederanteil von 25 Gew.-%, bezogen auf gesamten Sumpfstrom, abgezogen.
Die Sumpftemperatur betrug 190°C.
Die Destillationskolonne konnte unter diesen Bedingungen mehr als einen Monat lang störungsfrei betrieben werden.
Das über Kopf erhaltene Caprolactam war farblos und konnte problemlos polymerisiert werden.

### Vergleichsbeispiel 1

Es wurde wie in Beispiel 1 verfahren mit der Abweichung, dass die Sumpfabzugsmenge derart verringert wurde, dass der Hochsiederanteil 80 Gew.-%, bezogen auf gesamten Sumpfstrom, betrug.
Die Destillationsvorrichtung ließ sich zunächst betreiben.
Nach zwei Tagen hatte sich jedoch Feststoff im Sumpf gebildet, die die Leitungen verstopften und den weiteren betrieb der Destillationskolonne verhinderten.

### Vergleichsbeispiel 2

Es wurde wie in Beispiel 1 verfahren, mit der Abweichung, dass die Sumpftemperatur nur 150°C betrug.
Die Destillationsvorrichtung ließ sich zunächst betreiben.
Nach 12 Stunden hatte sich jedoch Feststoff im Sumpf gebildet, die die Leitungen verstopften und den weiteren betrieb der Destillationskolonne verhinderten.

## Patentansprüche

1. Verfahren zur Abtrennung von Hochsiedern aus einem Rohcaprolactam, das Hochsieder, Caprolactam und gegebenenfalls Leichtsieder enthält, und das erhalten wurde durch
a) Umsetzung von 6-Aminocapronitril mit Wasser zu einem Reaktionsgemisch
b) Abtrennung von Ammoniak und nicht umgesetztem Wasser aus dem Reaktionsgemisch unter Erhalt eines Rohcaprolactams,
**dadurch gekennzeichnet, dass** man
c) das Rohcaprolactam einer Destillationsvorrichtung zuführt unter Erhalt
eines ersten Teilstroms über Kopf als Produkt und
eines zweiten Teilstroms über Sumpf,
wobei man bei der Destillation
den Druck so einstellt, dass eine Sumpftemperatur von 170°C nicht unterschritten wird, und
den zweiten Teilstrom so einstellt, dass der Caprolactam-Gehalt des zweiten Teilstroms nicht weniger als 75 Gew.-%, bezogen auf den gesamten zweiten Teilstrom, beträgt,
wobei man den zweiten Teilstrom aus Schritt c) teilweise oder vollständig in Schritt a) zurückführt.

2. Verfahren nach Anspruch 1, wobei man Schritt a) in Gegenwart eines flüssigen Verdünnungsmittels durchführt.

3. Verfahren nach Anspruch 2, wobei man in Schritt b) das flüssige Verdünnungsmittel abtrennt.

4. Verfahren nach Ansprüchen 1 bis 3, wobei man die Abtrennung von Wasser in Schritt b) durch Überführung des Reaktionsgemisches in solche Bedingungen durchführt, dass das Reaktionsgemisch eine wasserreiche und eine wasserarme flüssige Phase bildet, wovon die wasserreiche Phase abgetrennt wird.

5. Verfahren nach den Ansprüchen 1 bis 4, wobei man zwischen den Schritten b) und c) Leichtsieder abtrennt.

6. Verfahren nach den Ansprüchen 1 bis 4, wobei man nach Schritt c) Leichtsieder abtrennt.

7. Verfahren nach Anspruch 5 oder 6, wobei man als Leichtsieder 6-Aminocapronitril abtrennt.

## Claims

1. A process for removing high boilers from crude caprolactam which comprises high boilers, caprolactam and in some cases low boilers, and which has been obtained by
a) reacting 6-aminocapronitrile with water to give a reaction mixture
b) removing ammonia and unconverted water from the reaction mixture to obtain crude caprolactam, which comprises
c) feeding the crude caprolactam to a distillation apparatus to obtain
a first substream via the top as a product and
a second substream via the bottom,
by setting the pressure in the distillation in such a way that the bottom temperature does not go below 170°C, and
adjusting the second substream in such a way that the caprolactam content of the second substream is not less than 75% by weight, based on the entire second substream, wherein the second substream from step c) is partly or fully recycled to step a).

2. The process according to claim 1, wherein step a) is carried out in the presence of a liquid diluent.

3. The process according to claim 2, wherein the liquid diluent is removed in step b).

4. The process according to any of claims 1 to 3, wherein the removal of water is carried out in step b) by transferring the reaction mixture into conditions such that the reaction mixture forms a high-water and a low-water liquid phase, of which the high-water phase is removed.

5. The process according to any of claims 1 to 4, wherein the low boilers are removed between steps b) and c).

6. The process according to any of claims 1 to 4, wherein low boilers are removed after step c).

7. The process according to claim 5 or 6, wherein the low boiler removed is 6-aminocapronitrile.

## Revendications

1. Procédé pour la séparation de la fraction à point d'ébullition élevé d'un caprolactame brut, qui contient une fraction à point d'ébullition élevé, du caprolactame et le cas échéant une fraction à bas point d'ébullition, et qui a été obtenu par
a) transformation de 6-aminocapronitrile avec de l'eau en un mélange réactionnel
b) séparation d'ammoniac et d'eau non transformée du mélange réactionnel avec obtention d'un caprolactame brut, **caractérisé en ce qu'**on
c) introduit le caprolactame brut dans un dispositif de distillation, en obtenant
un premier flux partiel via la tête comme produit et
un deuxième flux partiel via le fond,
où, dans la distillation
on règle la pression de manière telle que la température du fond ne descend pas au-dessous de 170°C et
on règle le deuxième flux partiel de manière telle que la teneur en caprolactame du deuxième flux partiel n'est pas inférieure à 75% en poids, par rapport à la totalité du deuxième flux partiel,
où on recycle le deuxième flux partie de l'étape c) en partie ou totalement dans l'étape a).

2. Procédé selon la revendication 1, où on réalise l'étape a) en présence d'un diluant liquide.

3. Procédé selon la revendication 2, où on sépare dans l'étape b) le diluant liquide.

4. Procédé selon les revendications 1 à 3, où on réalise la séparation de l'eau dans l'étape b) par transfert du mélange réactionnel dans des conditions telles que le mélange réactionnel forme une phase liquide riche en eau et une phase liquide pauvre en eau, dont la phase riche en eau est séparée.

5. Procédé selon les revendications 1 à 4, où on sépare une fraction à bas point d'ébullition entre les étapes b) et c).

6. Procédé selon les revendications 1 à 4, où on sépare une fraction à bas point d'ébullition après l'étape c).

7. Procédé selon la revendication 5 ou 6, où on sépare comme fraction à bas point d'ébullition du 6-aminocapronitrile.
